# EUROPEAN PATENT APPLICATION

(11) **EP 4 652 988 A1**
(43) Date of publication of application: **26.11.2025**
(21) Application number: 24744923.4
(22) Date of filing: 19.01.2024
(51) Int. Cl.: A61K 9/14, A61K 38/13, A61K 9/00, A61P 27/02

(54) **CYCLOSPORINE NANOMOLECULAR AGGREGATE, EYE-DROP COMPOSITION CONTAINING SAME, AND PREPARATION METHOD THEREFOR**

(30) Priority: 19.01.2023 KR 20230008013
(71) Applicant: Scai Therapeutics Co., Ltd., Seoul 06524 (KR)
(72) Inventor: KIM, Kyoung Hee, Daejeon 35249 (KR); HWANG, Si Ae, Daejeon 34052 (KR); KIM, Chul Hwan, Daejeon 35249 (KR)
(74) Representative: dompatent
(86) International application number: PCT/KR2024/000960
(87) International publication number: WO 2024/155144

(57) **Abstract**

The present invention relates to a cyclosporine nanomolecular association and an eye-drop composition containing the same. More specifically, the present invention relates to a cyclosporine nanomolecular association and an eye-drop composition containing the same, which has excellent storage stability and can be delivered to target tissues (cornea and conjunctiva) more efficiently at lower concentrations compared to conventional formulations.

## Description

### [Technical Field]

The present invention relates to a cyclosporine nanomolecular association, an eye-drop composition containing the same and a preparation method therefor. More specifically, the present invention relates to a cyclosporine nanomolecular association and an eye-drop composition containing the same, which has the effect of reducing side effects by including the above cyclosporine nanomolecular association, thereby reducing the cyclosporine dosage by 1/5 to 2/5 and reducing the used amount of excipients causing eye irritation symptoms by up to 90% compared to existing known products for preparing an eye-drop composition containing cyclosporine nanomolecular association, and a preparation method therefor.

### [Background Art]

There are various kinds of methods for treating dry eye, including artificial tears, cyclosporine eye drops, topical steroid eye drops, and autologous serum. Among these, cyclosporine is a peptide component derived from fungi that specifically inhibits the activity of T-helper cells in the ocular surface tissue, thereby blocking the production of IL-2, an immune-mediated inflammatory cytokine. Cyclosporine eye drops reduce the influx of lymphocytes into the lacrimal glands and effectively reduce apoptosis.

A representative drug of the prior art, 0.05% cyclosporine (Restasis^{®}, Allergan, Inc.), is a microemulsion type eye drop manufactured by mixing cyclosporine with polysorbate 80, castor oil, carbomer 1342, etc. An emulsion is a state in which two immiscible liquids are dispersed in another liquid in the form of small droplets at a certain ratio, and a microemulsion is a mixture of water-oil-surfactant, in which each component of the emulsion achieves phase equilibrium through thermodynamic self-assembly. Microemulsions are affected by various thermodynamic factors such as temperature and composition at equilibrium, and various particle shapes such as lamella sheets and hexahedral shapes coexist, and the particle sizes inside the microemulsion also vary from 67 to 2,489 nm. Due to these properties, the overall solution appears as a blue-tinged suspension. Therefore, cyclosporine prepared as a microemulsion is a suspended emulsion, which has the disadvantages of non-uniform particle size and ocular irritation. Additionally, Restasis^{®} has the disadvantage of low bioavailability, which requires it to reach the ocular surface.

### [Patent Document]

(Patent Document 1) US 8642556 B2

### [Disclosure]

### [Technical Problem]

In order to solve the above problems, the present invention provides a cyclosporine nanomolecular association and an eye-drop composition containing the same, which can reduce ocular pain by minimizing the amount of cyclosporine used, an active ingredient, by 1/5 to 2/5, while minimizing excipients that cause ocular pain by up to 90%, compared to the prior arts when preparing an eye-drop composition.

In addition, the present invention provides a method for preparing the cyclosporine nanomolecular association and the eye-drop composition comprising the same.

### [Technical Solution]

The present invention provides a molecular association in which cyclosporine is physically bound, wherein the molecular association in the composition has an aggregated structure when the molecular association is prepared as a composition containing water.

In addition, according to one embodiment of the present invention, it can provide a molecular association having an average particle diameter of 1.0 to 10 nm or less.

In addition, according to one embodiment of the present invention, it can provide a molecular association in which the total amount of related substances is 1.5% or less of the initial amount after 6 months under long-term storage conditions at a temperature of 5°C±3°C.

In addition, according to one embodiment of the present invention, it can provide a molecular association in which the total amount of related substances is 1.5% or less of the initial amount after 6 months under accelerated conditions of 25°C±2°C/70%±5%.

In addition, the present invention provides a composition for preparing an eye drops comprising the molecular association, a surfactant, a solubilizer, and purified water.

In addition, according to one embodiment of the present invention, it can provide a composition for preparing an eye drop, wherein the molecular association is contained in an amount of 0.001 to 0.04 wt% based on the total content of the composition.

In addition, according to one embodiment of the present invention, it can provide a composition for preparing an eye drop, wherein the molecular association is contained in an amount of 0.001 to 0.02 wt% based on the total content of the composition.

In addition, according to one embodiment of the present invention, it can provide a composition for preparing an eye drop, wherein a surfactant is contained in an amount of 0.001 to 0.5 wt% based on the total content of the composition.

In addition, according to one embodiment of the present invention, it can provide a composition for preparing an eye drop, wherein a surfactant is contained in an amount of 0.01 to 0.1 wt% based on the total content of the composition.

In addition, according to one embodiment of the present invention, it can provide a composition for preparing an eye drop, wherein a solubilizer is contained in an amount of 0.001 to 0.1 wt% based on the total content of the composition.

In addition, according to one embodiment of the present invention, it can provide a composition for preparing an eye drop, wherein the surfactant is at least one selected from the group consisting of: Polysorbate 20, Polysorbate 40, Polysorbate 60, Polysorbate 80, alcohol, amine oxide, block polymer, carboxylate alcohol, alkylphenol ethoxylate carboxylic acid/fatty acid, ethoxylate alcohol, ethoxylate alkylphenol, ethoxylate aryl phenol, ethoxylated fatty acid, ethoxylate, fatty acid ester or oil, fatty acid ester, fatty acid methyl ester ethoxylate, glycerol ester, glycol ester, lanolin derivatives, lecithin, lecithin derivatives, lignin, lignin derivatives, methyl ester, monoglyceride, monoglyceride derivatives, polyethylene glycol, polymeric surfactant, propoxylate and ethoxylate fatty acids, alcohol or alkyl phenol, protein derived surfactant, sarcosine derivatives, sorbitan derivatives, sucrose, sucrose derivatives, glucose esters, and glucose ester derivatives.

In addition, according to one embodiment of the present invention, it can provide a composition for preparing an eye drop, wherein the solubilizer is at least one selected from the group consisting of: castor oil, canola oil, alkyl (C12-15) benzoate, almond oil, aluminum monostearate, cetostearyl alcohol, cholesterol, coconut oil, cyclomethicone, dimethicone, ethylene glycol stearate, glycerin, glyceryl monooleate, glyceryl monostearate, isopropyl myristate, isopropyl palmitate, hydrogenated lanolin, lecithin, mineral oil, myristyl alcohol, octyldodecanol, oleyl alcohol, oleyl oleate, petrolactum, polydecane, propylene glycol dilaurate, propylene glycol monolaurate, safflower oil, soybean oil, sunflower oil, wax, xylitol and zinc acetate.

In addition, according to one embodiment of the present invention, it can provide an eye-drop composition further comprising at least one selected from the group consisting of a viscosity adjusting agent, an osmotic pressure adjusting agent and a pH adjusting agent.

In addition, according to one embodiment of the present invention, it can provide an eye-drop composition, wherein the viscosity adjusting agent is contained in an amount of 0.001 to 0.5 wt% based on the total content of the composition.

In addition, according to one embodiment of the present invention, it can provide an eye-drop composition, wherein the osmotic pressure adjusting agent is contained in an amount of 0.5 to 10.0 wt% based on the total content of the composition.

In addition, according to one embodiment of the present invention, it can provide an eye-drop composition, wherein the content of the viscosity adjusting agent is 0.5 to 2.5 times the content of the molecular association.

In addition, according to one embodiment of the present invention, it can provide an eye-drop composition, wherein the viscosity adjusting agent is at least one selected from the group consisting of: acacia, agal, aramic acid, alginic acid, aluminum monostearate, attapulgite, bentonite, carbomer, carboxymethylcellulose calcium, carboxymethylcellulose sodium, carmellrose, carrageenan, cellulose, microstalline, cetostearyl alcohol, chitosan, corn syrup, cyclomethicone, dextrin, egg phospholipids, ethyl cellulose, gelatin, magnesium aluminum silicate, maltitol solution, maltodextrin, medium-chain triglycerides, methylcellulose, pectin, polycarbophil, polydextrose, polyethylene oxide, polyvinyl alcohol, potassium alginate, povidone, propylene glycol alginate, pullulan, silica dental-type, silica hydrophobic colloidal, silicon dioxide, silicon dioxide colloidal, sodium alginate, sorbitan monolaurate, sorbitan monopalmitate, sorbitan monostearate, sorbitan sesquioleate, sorbitan trioleate, starch corn, starch hydroxypropyl corn, starch pregelatinized hydroxypropyl corn, starch pea, starch hydroxypropyl pea, starch pregelatinized hydroxypropyl pea, starch potato, gellan gum, glyceric behenate, glyceric dibehenate, guar gum, hydroxyethyl cellulose, hydroxypropyl cellulose, hydromellose, isomalt, alpha-lactalbumin, kaolin, starch hydroxypropyl potato, starch pregelatinized hydroxypropyl potato, starch tapioca, starch wheat, sucrose, sucrose palmitate, tragacanth, vitamin E propylene glycol succinate, and xanthan gum.

In addition, according to one embodiment of the present invention, it can provide an eye-drop composition, wherein the osmotic pressure adjusting agent is at least one selected from the group consisting of glycerin, sorbitol, mannitol, dextrose and sucrose.

In addition, according to one embodiment of the present invention, it can provide an eye-drop composition, wherein the pH adjusting agent is at least one selected from the group consisting of: acetic acid, adipic acid, ammonia solution, ammonium carbonate, ammonium chloride, ammonium phosphate, boric acid, calcium carbonate, calcium hydroxide, calcium lactate, calcium phosphate, citric acid, diethanolamine, fumaric acid, glycine, hydrochloric acid, alpha-lactalbumin, lactic acid, lysine hydrochloride, malic acid, maleic acid, methionine, monoethanolamine, monosodium glutamate, nitric acid, phosphoric acid, potassium citrate, potassium hydroxide, potassium metaphosphate, potassium phosphate, propionic acid, racemethionine, sodium acetate, sodium bicarbonate, sodium borate, sodium carbonate, sodium citrate, sodium hydroxide, sodium lactate solution, sodium phosphate dibasic, sodium phosphate monobasic, succinic acid, sulfuric acid, tartaric acid and trolamine.

In addition, according to one embodiment of the present invention, it can provide an eye-drop composition characterized in that the molecular association is contained in an amount of 0.001 to 0.04 wt% based on the total content of the composition, and the total content change for 6 months under long-term storage conditions of 25°C±2°C/40%±5% is 90.0 to 110.0%.

In addition, according to one embodiment of the present invention, it can provide an eye-drop composition characterized in that the molecular association is contained in an amount of 0.001 to 0.04 wt% based on the total content of the composition, and the total content change for 6 months under intermediate conditions of 30°C±2°C/65%±5% is 90.0 to 110.0%.

In addition, according to one embodiment of the present invention, it can provide an eye-drop composition characterized in that the molecular association is contained in an amount of 0.001 to 0.04 wt% based on the total content of the composition, and the total content change for 6 months under acceleration conditions of 40°C±2°C/25%±5% is 80.0 to 110.0%.

In addition, according to one embodiment of the present invention, it can provide an eye-drop composition, wherein the exposure amount to cornea and conjunctiva is 3 times or more when administered to the eye.

In addition, according to one embodiment of the present invention, it can provide an eye-drop composition, wherein the amount of blood releasing out to the whole body other than the eye tissue is 0.02% or less of the total eye drop dosage when administered to the eye.

The present invention provides a method for preparing a molecular association in which cyclosporine is physically bound, comprising the steps of: 1) pouring a cyclosporine solution into silica and then repeatedly releasing cyclosporine; 2) filtering the released solution and then concentrating the filter filtrate through vacuum distillation; 3) filtering the concentrated residual liquid and then crystallizing it; and 4) filtering, washing, and drying the crystallized solid.

### [Advantageous Effects]

The present invention reduces the used amount of excipients causing eye irritation symptoms by up to 90% while reducing the amount of cyclosporine active ingredient by 1/5 to 2/5, so that it can be efficiently delivered to target tissues (cornea, conjunctiva) at a lower concentration. In addition, it has the advantage that the amount of blood releasing out to the whole body other than the target tissue of the drug, the eye tissue, is low at 0.02% or less of the total eye drop dosage for both single and multiple eye drop administration. In addition, it has storage stability that realizes and maintains a very small particle size of 2 nm.

### [Description of Drawings]

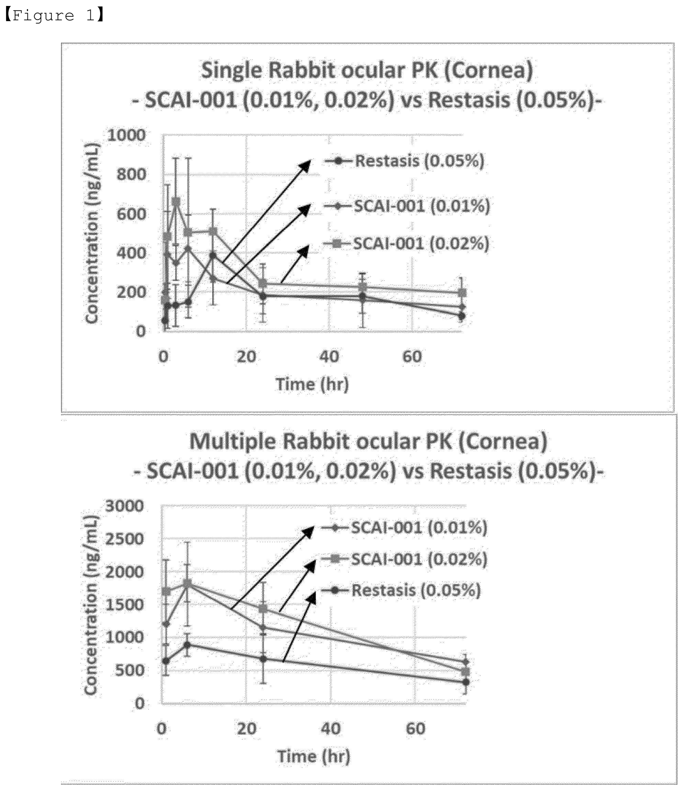

FIG. 1 is a view illustrating the results of a pharmacodynamic comparison of the distribution of the main drug ingredient, cyclosporine A, in the cornea over time after single or multiple eye drop administration to the eye of a rabbit of Restasis (0.05%), a comparative preparation from Allergan company, and the eye drop of Example 3 of the present invention (0.01% and 0.02%).

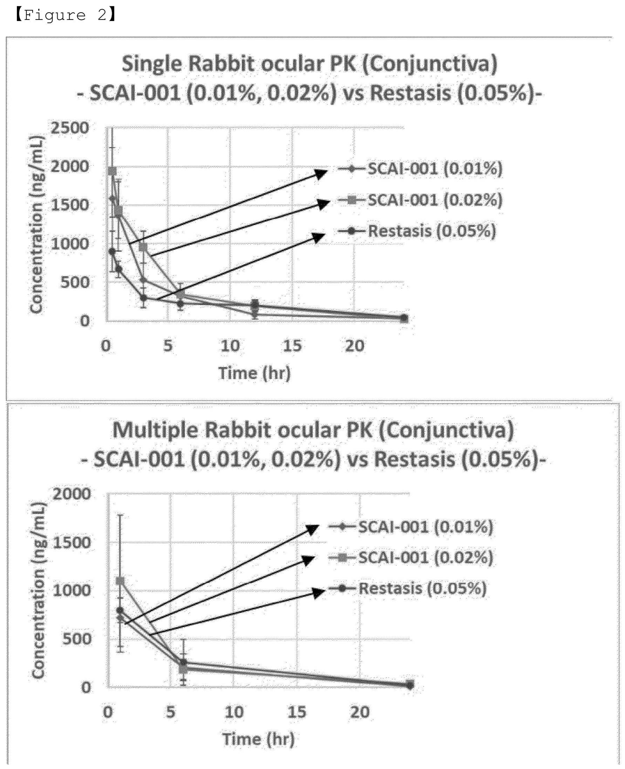

FIG. 2 is a view illustrating the results of a pharmacodynamic comparison of the distribution of the main drug ingredient, cyclosporine A, in the conjunctiva over time after single or multiple eye drop administration to the eye of a rabbit of Restasis (0.05%), a comparative preparation from Allergan company, and the eye drop of Example 3 of the present invention (0.01% and 0.02%).

### [Best Mode]

The present invention presents a cyclosporine nanomolecular association that can be efficiently delivered to target tissues (cornea, conjunctiva) at a lower concentration by reducing the amount of cyclosporine active ingredient by 1/5 to 2/5 and reducing the used amount of excipients causing eye irritation symptoms by up to 90% compared to the prior art. In addition, the present invention presents a cyclosporine nanomolecular association having the advantage in that the amount of blood releasing out to the whole body other than the target tissue of the drug, the eye tissue, is 0.02% or less of the total eye drop dosage when administered to the eye for both single and multiple eye drop administrations. Furthermore, the present invention presents a cyclosporine nanomolecular association having storage stability that realizes and maintains a very small particle size of 2 nm.

The present invention will be described in more detail below.

### Terms

In the present invention, the term "eye drop" means administering a liquid preparation in the form of a drop onto the outer surface of the eye.

In the present invention, the term "eye drops" refers to a pharmaceutical liquid preparation that is administered in the form of a drop on the outer surface of the eye and exhibits a local effect on the posterior segment of the eye.

In the present invention, the term "composition for preparing an eye drops" refers to an intermediate composition prepared before preparing an eye-drop composition, which additionally contains a surfactant, a solubilizer, water, etc. in addition to a molecular association. An eye drop adjusting agent can be made by additionally including a viscosity adjusting agent, an osmotic pressure adjusting agent, a pH adjusting agent, etc.

In the present invention, the term "exposure amount" refers to the amount of the active ingredient of cyclosporine that reaches cornea and conjunctiva.

In the present invention, the term "release" means the amount of cyclosporine that does not remain in the target area, such as the cornea and conjunctiva of the eye, but flows into the blood vessels or bloodstream.

In the present invention, the term "related substance" refers to a type of impurity that may be created during the process of synthesizing raw materials or manufacturing finished pharmaceutical products. These related substances must be contained in the final pharmaceutical product in levels below the standard and are substances used to verify quality.

In the present invention, the terms "patient", "subject", "individual" and the like are used interchangeably herein and refer to any animal, or cells thereof, either in vitro or in situ, that can be amenable to the methods described herein. In certain non-limiting embodiments, the patient, subject or individual is a human.

In the present invention, the term "composition" means a mixture of at least one compound of the present invention and other chemical components such as carriers, stabilizers, diluents, dispersants, suspending agents, thickening agents, and/or excipients.

In the present invention, the terms "effective amount", "pharmaceutically effective amount" and "therapeutically effective amount" refer to an amount that is non-toxic but is sufficient to provide the desired biological result. The above results may be a reduction and/or alleviation of the signs, symptoms, or causes of a disease, or any other desired alteration of a biological system. The appropriate therapeutic amount in any individual case can be determined by the skilled person using routine experimentation.

In the present invention, the term "efficacy" refers to the maximum effect (Emax) achieved within an analytical method.

### Cyclosporine nanomolecular association of the present invention

The present invention provides a molecular association in which cyclosporine is physically bound, wherein the molecular association in the composition has an aggregated structure when the molecular association is prepared as a composition containing water.

In the present invention, the average particle diameter of the molecular association may be 1.0 to 10 nm or less, preferably 1.5 nm or more, 2.0 nm or more, and 7.0 nm or less, 5.0 nm or less, or 3.0 nm or less. The average particle diameter of the molecular association can be measured through a diffraction experiment, and preferably can be measured using a Zetasizer or Small Angle Neutron Scattering (SANS). Images can also be measured using Transmission Electron Microscopy. If the average particle diameter of the molecular association exceeds 10 nm, there is a problem that dispersibility decreases and transparency and transmittance decrease. In addition, there is no particular limitation on the lower limit of the average particle diameter of the structure, but a structure having a diameter of approximately 0.5 nm or more can be used.

In addition, the molecular association according to the present invention has excellent storage stability. Unlike general nano-sized dispersed particles that are easily exposed to aggregation or Ostwald ripening phenomenon and thus have low storage stability, the molecular association and composition of the present invention have excellent stability because the amount of change in the amount of related substance is small under long-term storage conditions, intermediate storage conditions, or accelerated conditions. The change rate refers to the average change rate calculated by averaging the monthly change rates up to the above period.

As described above, it can be seen that the stability of the molecular association according to the present invention is excellent.

Specifically, the molecular association may have a total related substance amount of 1.5% or less compared to the initial amount after 6 months under long-term storage conditions at a temperature of 5°C±3°C, preferably 1.0% or less, and more preferably 0.5% or less.

In addition, the molecular association may have a total related substance amount of 1.5% or less compared to the initial amount after 6 months under accelerated conditions of 25°C±2°C/70%±5%, preferably 1.0% or less, and more preferably 0.5% or less.

### Composition for preparing an eye drops of cyclosporine nanomolecular association of the present invention

The present invention provides a composition for preparing an eye drops, which further comprises a surfactant, a solubilizer and purified water in the molecular association. This composition for preparing an eye drops can be supplied to a user who wants to manufacture an eye drops, and then additional components of the eye-drop composition described below can be added to prepare the eye-drop composition.

The molecular association may be included in an amount of 0.001 to 0.04 wt% based on the total content of the composition for preparing an eye drops, preferably 0.005 wt% or more, more preferably 0.01 wt% or more, and preferably 0.03 wt% or less, more preferably 0.02 wt% or less.

In the composition for preparing an eye drops of the present invention, as the surfactant, at least one selected from the group consisting of Polysorbate 20, Polysorbate 40, Polysorbate 60. Polysorbate 80, alcohol, amine oxide, block polymer, carboxylate alcohol, alkylphenol ethoxylate carboxylic acid/fatty acid, ethoxylate alcohol, ethoxylate alkylphenol, ethoxylate aryl phenol, ethoxylated fatty acid, ethoxylate, fatty acid ester or oil, fatty acid ester, fatty acid methyl ester ethoxylate, glycerol ester, glycol ester, lanolin derivatives, lecithin, lecithin derivatives, lignin, lignin derivatives, methyl ester, monoglyceride, monoglyceride derivatives, polyethylene glycol, polymeric surfactant, propoxylate and ethoxylate fatty acids, alcohol or alkyl phenol, protein derived surfactant, sarcosine derivatives, sorbitan derivatives, sucrose, sucrose derivatives, glucose ester, and glucose ester derivatives may be additionally included.

In the composition for preparing an eye drops of the present invention, a significantly smaller amount of surfactant can be used compared to the conventional composition for preparing an eye drops. Specifically, the surfactant may be included in an amount of 0.01 to 0.1 wt% based on total content of the composition, preferably 0.03 wt% or more, more preferably 0.05 wt% or more, and preferably 0.09 wt% or less, more preferably 0.08 wt% or less.

If the content of the surfactant is higher than the above range, there is a problem that the burning sensation becomes severe and the toxicity increases when administered to cornea and conjunctiva, and if it is lower, there is a problem that it becomes difficult to maintain the size of the prepared droplets and the permeability into cells decreases.

In the composition for preparing an eye drops of the present invention, the above solubilizer may additionally include at least one selected from the group consisting of: castor oil, canola oil, alkyl (C12-15) benzoate, almond oil, aluminum monostearate, cetostearyl alcohol, cholesterol, coconut oil, cyclomethicone, dimethicone, ethylene glycol stearate, glycerin, glyceryl monooleate, glyceryl monostearate, isopropyl myristate, isopropyl palmitate, hydrogenated lanolin, lecithin, mineral oil, myristyl alcohol, octyldodecanol, oleyl alcohol, oleyl oleate, petrolactum, polydecane, propylene glycol dilaurate, propylene glycol monolaurate, safflower oil, soybean oil, sunflower oil, wax, xylitol and zinc acetate.

In the composition for preparing an eye drop of the present invention, a significantly smaller amount of a solubilizer can be used compared to the conventional composition for preparing an eye drops. Specifically, the solubilizer may be included in an amount of 0.001 to 0.1 wt% based on the total content of the composition, preferably 0.01 wt% or more, more preferably 0.02 wt% or more, and preferably 0.05 wt% or less, more preferably 0.03 wt% or less.

If the content of the solubilizer is higher than the above range, there is a problem of blurred vision and burning (stinging) sensation, and if it is lower, there is a problem of reduced corneal epithelial permeability.

### Eye-drop composition of cyclosporine nanomolecular association of the present invention

The present invention provides an eye-drop composition further comprising at least one selected from the group consisting of a viscosity adjusting agent, an osmotic pressure adjusting agent and a pH adjusting agent in the composition for preparing an eye drops.

In the eye-drop composition of the present invention, the osmotic pressure adjusting agent may additionally include at least one selected from the group consisting of glycerin, sorbitol, mannitol, dextrose, and sucrose.

In the eye-drop composition of the present invention, the osmotic pressure adjusting agent may be included in an amount of 0.5 to 10 wt% based on the total content of the composition.

In the eye-drop composition of the present invention, the above viscosity adjusting agent may additionally include at least one selected from the group consisting of: acacia, agal, aramic acid, alginic acid, aluminum monostearate, attapulgite, bentonite, carbomer, carboxymethylcellulose calcium, carboxymethylcellulose sodium, carmellrose, carrageenan, cellulose, microstalline, cetostearyl alcohol, chitosan, corn syrup, cyclomethicone, dextrin, egg phospholipids, ethyl cellulose, gelatin, magnesium aluminum silicate, maltitol solution, maltodextrin, medium-chain triglycerides, methylcellulose, pectin, polycarbophil, polydextrose, polyethylene oxide, polyvinyl alcohol, potassium alginate, povidone, propylene glycol alginate, pullulan, silica dental-type, silica hydrophobic colloidal, silicon dioxide, silicon dioxide colloidal, sodium alginate, sorbitan monolaurate, sorbitan monopalmitate, sorbitan monostearate, sorbitan sesquioleate, sorbitan trioleate, starch corn, starch hydroxypropyl corn, starch pregelatinized hydroxypropyl corn, starch pea, starch hydroxypropyl pea, starch pregelatinized hydroxypropyl pea, starch potato, gellan gum, glyceric behenate, glyceric dibehenate, guar gum, hydroxyethyl cellulose, hydroxypropyl cellulose, hydromellose, isomalt, alpha-lactalbumin, kaolin, starch hydroxypropyl potato, starch pregelatinized hydroxypropyl potato, starch tapioca, starch wheat, sucrose, sucrose palmitate, tragacanth, vitamin E propylene glycol succinate, and xanthan gum.

In the eye-drop composition of the present invention, the viscosity adjusting agent may be included in an amount of 0.001 to 0.5 wt% based on the total content of the composition. If the content of the viscosity adjusting agent is included in a higher amount than the above range, problems such as eye irritation, pain or discomfort, and eyelid crusting may occur, and if the content is included in a lower amount, a problem of dry eye may occur.

In addition, the content of the viscosity adjusting agent may be 0.5 to 2.5 times the content of the molecular association. When the content ratio of the viscosity adjusting agent and the molecular association satisfies the above range, there is an advantage in that no burning sensation occurs.

In the eye-drop composition of the present invention, the above pH adjusting agent may additionally include at least one selected from the group consisting of: acetic acid, adipic acid, ammonia solution, ammonium carbonate, ammonium chloride, ammonium phosphate, boric acid, calcium carbonate, calcium hydroxide, calcium lactate, calcium phosphate, citric acid, diethanolamine, fumaric acid, glycine, hydrochloric acid, alpha-lactalbumin, lactic acid, lysine hydrochloride, malic acid, maleic acid, methionine, monoethanolamine, monosodium glutamate, nitric acid, phosphoric acid, potassium citrate, potassium hydroxide, potassium metaphosphate, potassium phosphate, propionic acid, racemethionine, sodium acetate, sodium bicarbonate, sodium borate, sodium carbonate, sodium citrate, sodium hydroxide, sodium lactate solution, sodium phosphate dibasic, sodium phosphate monobasic, succinic acid, sulfuric acid, tartaric acid and trolamine.

In the eye-drop composition of the present invention, the pH adjusting agent may be included in an appropriate amount for pH adjustment.

Even if additional components as described above are added, the eye-drop composition of the present invention may contain molecular associations in an amount of 0.001 to 0.04 wt% based on the total content of the composition, preferably 0.005 wt% or more, more preferably 0.01 wt% or more, and preferably 0.03 wt% or less, more preferably 0.02 wt% or less.

In addition, the molecular association included in the eye-drop composition according to the present invention has excellent storage stability. Unlike general nano-sized dispersed particles that are easily exposed to aggregation or Ostwald ripening phenomenon and thus have low storage stability, the molecular association contained in the eye-drop composition according to the present invention and composition have excellent stability because the amount of change in the molecular association is small under long-term storage conditions, intermediate storage conditions, or accelerated conditions. The above change rate refers to the average change rate calculated by averaging the monthly change rates up to the above period.

As described above, it can be seen that the stability of the molecular association according to the present invention is excellent.

Specifically, in the eye-drop composition, the molecular association included in the eye-drop composition is included in an amount of 0.001 to 0.04 wt% based on the total content of the composition, and the total content change for 6 months under long-term storage conditions of 25°C±2°C/40%±5% may be 80.0 to 110.0%, preferably 90.0 to 110.0%.

In addition, in the eye-drop composition, the molecular association included in the eye-drop composition is included in an amount of 0.001 to 0.04 wt% based on the total content of the composition, and the total content change for 6 months under intermediate conditions of 30°C±2°C/65%±5% can be 80.0 to 110.0%, and preferably 90.0 to 110.0%.

In addition, in the eye-drop composition, the molecular association included in the eye-drop composition is included in an amount of 0.001 to 0.04 wt% based on the total content of the composition, and the total content change for 6 months under 40°C±2°C/25%±5% acceleration conditions may be 80.0 to 110.0%, preferably 90.0 to 110.0%.

The eye-drop composition according to the present invention comprises a molecular association of cyclosporine, an active ingredient, in an amount much smaller than that of the eye-drop compositions of the prior art, that is, a lower concentration, of 0.001 to 0.04 wt% based on the total content of the composition, but the active ingredient, cyclosporine, can be efficiently present in cornea and conjunctiva. Accordingly, when the eye-drop composition is administered to the eye, the exposure amount to the cornea and conjunctiva may be at least 2 times, preferably at least 3 times, more preferably at least 4 times, and most preferably at least 5 times.

In addition, in the eye-drop composition according to the present invention, when the eye-drop composition is administered to the eye, the amount of blood releasing out to the whole body other than the eye tissue may be 0.02% or less, preferably 0.01% or less, and more preferably 0.005% or less of the total eye drop dosage.

### Method for preparing cyclosporine nanomolecular association and eye-drop composition of the present invention

According to one embodiment of the present invention, the molecular association of cyclosporine A can be prepared by applying shear stress to a solution containing cyclosporine A, a precursor of the molecular association.

The shear stress applied to the solution containing cyclosporine A, a precursor of the above molecular association, may be a mechanical shear stress.

The mechanical shear stress may be applied by passing the solution through a column or filter paper filled with silica. The mechanical shear stress is specifically described below.

More specifically, the present invention provides a method for preparing a molecular association in which cyclosporine is physically bound, comprising the steps of:
1) pouring a cyclosporine solution into silica and then repeatedly releasing cyclosporine; 2) filtering the released solution and then concentrating the filter filtrate through vacuum distillation; 3) filtering the concentrated residual liquid and then crystallizing it; and 4) filtering, washing, and drying the crystallized solid.

First, the method for preparing a molecular association includes 1) a step of pouring a cyclosporine solution into silica and then repeatedly releasing cyclosporine.

According to one embodiment of the present invention, the mechanical shear stress may be applied by passing a solution containing cyclosporine A, a precursor of the molecular association, through a column filled with silica. When the solution containing cyclosporine A passes through a column filled with silica or the like, the precursor of the molecular association, cyclosporine A, is subjected to very high shear stress by passing through a physically narrow area.

The silica may be spherical or angular, but is not limited to its shape.

The average particle size of the silica may be 1.0 to 50 µm, specifically, may be 1.5 µm or more, 2 µm or more, and 40 µm or less, 30 µm or less, 20 µm or less, 10 µm or less, or 5 µm or less. When the size of the silica is less than 1.0 µm or exceeds 50 µm, even if the solution containing cyclosporine A passes through a column filled with silica, no shear stress is applied, so there may be no change in the molecular association.

A negative pressure of 0.1 bar to 1.0 bar or 0.2 bar to 0.9 bar can be applied to the lower portion of the column filled with the silica. When the negative pressure applied to the lower portion of the column filled with the silica is less than 0.1 bar, the time required for the solution containing cyclosporine A to pass through the column increases, so the time required for the production of the molecular association of cyclosporine A according to the present invention may be delayed. In addition, when the negative pressure applied to the lower portion of the column filled with the silica exceeds 1.0 bar, the time required for the solution containing cyclosporine A to pass through the column is reduced, so that the time required for the production of the molecular association of cyclosporine A according to the present invention can be shortened. However, since additional pump equipment is required, the manufacturing cost may increase.

According to another embodiment of the present invention, the mechanical shear stress may be applied by passing the solution containing cyclosporine A through one or more filter papers. When passing through one or more of the filter papers, cyclosporine A, a precursor of the molecular association, is subjected to very high shear stress by passing through a physically narrow region.

The filter paper may be one filter paper or two or more filter papers. When the filter paper is two or more filter papers, the filter papers can be arranged in a stacked manner. When the filter paper is two or more filter papers, a higher shear stress can be provided than a single filter paper.

The pore size of the filter paper may be 0.1 to 5.0 microns or 0.3 to 4.5 microns. When the pore size of the filter paper is less than 0.1 micron, the amount of the solution containing the bile acid passing through or filtering the filter paper is too small, so that the production speed of the molecular association of cyclosporine A according to the present invention may decrease, and when the pore size of the filter paper exceeds 5.0 micron, the solution containing the bile acid may simply pass through the filter paper, so that shear stress may not be effectively applied.

Next, the method for preparing a molecular association according to the present invention includes 2) a step of filtering the released solution and then concentrating the filter filtrate through vacuum distillation. The filtering, vacuum distillation, and concentration methods can be used without special restrictions as long as they are commonly used in the industry.

Next, the method for preparing a molecular association according to the present invention includes 3) a step of filtering the concentrated residual liquid and then crystallizing it. The filtering and crystallization methods can be used without special restrictions as long as they are commonly used in the industry.

Next, the method for preparing a molecular association according to the present invention includes 4) a step of filtering, washing, and drying the crystallized solid. The above filtration, washing and drying can be used without special restrictions as long as they are commonly used in the industry.

Hereinafter, the present invention will be described in more detail through examples of the present invention. It will be understood that the present invention is not limited to these examples.

### [Example]

### Example 1. Preparation of cyclosporine nanomolecular association(SCAI-001)

1) 0.33 kg of cyclosporineA API (hereinafter referred to as CsA) was weighed.
2) 22.38 kg of 94.5% ethanol (hereinafter referred to as ethanol) was weighed separately.
3) The ethanol of the above 2) was added to the CSA of the above 1) and stirred for 1 hour using a stirrer to prepare a CsA solution in advance.
4) 16.70 kg of ethanol was weighed separately.
5) Grace SYLOID 244FP was sieved using a mesh screen of 425µm.
6) 1.67 kg of 425µm Silica was weighed and then the ethanol from 4) was added thereto and sufficiently wetted with a spatula.
7) 33.40 kg of 94.5% EtOH was weighed separately.
8) A Buchner funnel was placed on the Erlenmeyer flask and a 1.0 µm paper filter was placed on the funnel.
9) The 1.0 µm paper filter was moistened with EtOH, and the vacuum pump was operated to adsorb it to the bottom of the funnel, after which the wetted silica of the above 6) was slowly poured.
10) When the silica supernatant remains at about 0.5 cm, the vacuum pump was stopped, the filter filtrate was discarded, and a new flask was replaced.
11) The CSA solution prepared in the above 3) was slowly poured over the packed silica in several portions to prevent the silica from being destroyed.
12) When about 0.5 cm of silica supernatant remains, the EtOH prepared in 7) was poured in two portions of 16.70 kg each.
13) The filter filtrate of the above 12) was filtered using a 0.45 µm membrane filter (PVDF).
14) The filtered filtrate was concentrated to 2.34 kg using a rotary depressurizer (28°C, 80 to 180 rpm, 20 mbar).
15) 0.33 kg of 94.5% EtOH was weighed twice separately.
16) 2.34 kg of the concentrate was filtered using a 0.22 µm membrane filter (PVDF). The CSA concentrate on the filter was washed with EtOH prepared in 15).
17) 0.33 kg of 94.5% EtOH was weighed separately.
18) 100.20 kg of purified water was prepared by filtering through a 0.22 µm (PVDF) membrane.
19) By preparing an overhead stirrer and an impeller, preparation for crystallization was made, and the concentrate of the above 16) was slowly dropped into the purified water of the above 18) for 40 minutes.
20) When dropwise addition was completed, the concentrate of 16) above was washed with the prepared EtOH of 17) above and then added.
21) The mixture of the above 20) was stirred for 2 hours and allowed to settle for 1 hour.
22) Purified water was filtered through a 0.22 µm (PVDF) membrane and 5.01 kg was prepared separately.
23) The mixture of the above 21) was poured using a glass filter (G3) and the crystallized solid was filtered.
24) The prepared purified water of the above 22) was added to the mixture of the above 21) and washed, and the crystallized solid was washed away.
25) The filtered crystallized solid was filtered under reduced pressure by operating a vacuum pump for about 1 hour and then dried in a vacuum dryer (50°C, -0.1 bar).
26) The moisture content was checked using K.F. and if it was less than 2.5%, a solubility test was conducted.
27) When the value of the above 26) was satisfied, it was filtered using a 500 µm mesh screen sieve, and cyclosporine nanomolecular association (SCAI-001) was obtained.

The specific results obtained are as shown in Table 1 below.

**[Table 1]**

| **Batch No.** | **Sample amount (g)** | **Yield (%)** | **Particle size (nm)** | **Section** |
|---|---|---|---|---|
| CsA2107JJ12-42 | 110.16 (10gx17 times) | 65% | 2.0±0.15 | Stability test sample |
| CsA2107JJ13-11 | 48.1 (10gx5 times) | 96% | 2.0±0.20 | |
| CsA2107JJ13-12 | 42.59 (10gx5 times) | 85% | 2.0±0.25 | |
| CsA2107JJ13-21 | 38.56 (10gx5 times) | 77% | 2.0±0.20 | |
| **CsA2107JJ14** | **239.41** | **75** | | |

The test results of the laboratory-manufactured samples were as shown in Table 2 below.

**[Table 2]**

| Test item | Test criteria | Test result |
|---|---|---|
| Appearance | White or almost white powder | White or almost white powder |
| Verification test | The main peak retention time of the standard solution and the test solution is the same | The main peak retention time of the standard solution and the test solution is the same |
| Content test | 97.0 ~ 101.5 %(As a dry matter) | 98.94 % ( As a dry matter) |
| Related substance | Individual unknown related substance: 0.7 % or less Total related substance: 1.5 % or less | Individual unknown related substance: 0.07%, 0.12% Total related substance: 0.71% |
| Loss on drying | 2.0 % or less | 1.06% |

### Example 2. Preparation of composition for preparing an eye drops containing cyclosporine nanomolecular association(SCAI-001)

1) After weighing 40 g of cyclosporine and 40 g of castor oil in an STS reactor, 8 kg of 95% ethanol (hereinafter referred to as ethanol) was added and dissolved for 1 hour while maintaining the internal temperature at 10 to 25°C. Separately, 8 kg of ethanol and 400 g of silica were weighed into a G/L reactor and stirred uniformly for 30 minutes. The wetted silica from the G/L reactor was slowly filtered through a Nutsche filter until ethanol reached 1 to 2 cm on the surface to form a silica packing layer. The filtrate was then discarded.
2) The solution from the STS reactor is filtered through a Nutsche filter with a packed layer, then filtered through a housing filter (10 inch, 0.45 µm PES) and transferred to the G/L reactor of 1), and the filter is washed with 12 kg of ethanol and then transferred.
3) 60 kg of ethanol and 0.16 kg of polysorbate 80 were added to the G/L reactor of 2) and stirred for 1 hour.
4) Separately, 300 kg of PW (purified water) was added to the STS reactor to prepare it, and then the reaction solution of the G/L reactor of the above 3) was added dropwise for more than 1 hour. After the dropwise addition was completed, it was stirred for 30 minutes.
5) The reaction solution of the STS reactor was first concentrated under reduced pressure at an internal temperature of 30°C or lower. After the primary concentration was completed, the reaction solution from the STS reactor was filtered through a housing filter (10 inch, 0.45 µm PES) to remove precipitates. The filtrate filtered through the housing filter was concentrated for a second time at an internal temperature of 35°C or lower. After the secondary concentration was completed, the secondary concentrate was tested for cyclosporine concentration and residual ethanol (cyclosporine concentration: 1.0 mg/mL ± 10%, residual ethanol 5000 ppm or less). After the process inspection was completed, the concentrate from the STS reactor was filtered through a housing filter (10 inch, 0.2 µm PES), divided into 22.78 kg portions and packaged in LDPE sterile collection bottles to obtain a composition for preparing an eye drops containing SCAI-001.

### Example 3. eye-drop composition containing cyclosporine nanomolecular association(SCAI-001)

### 1) Preparation

A formulation study of the composition for eye drop containing SCAI-001 as an active ingredient was conducted, and stability samples and clinical samples were produced through consignment manufacturing.

A formulation study was completed using only the auxiliary components used in Restasis, a product of Allergan company.

Using the molecular association and composition for preparing an eye drops obtained in Example 1 and Example 2 above, the composition for an eye-drops was prepared as shown in Table 3 below.

**[Table 3]**

| | Composi tion 1 (0.02 %) | Composi tion 2 (0.02 %) | Composit ion 3 (0.02 %) | Composi tion 4 (0.02 %) | Composi tion 5 (0.02 %) | Composi tion 6 (0.01 %) |
|---|---|---|---|---|---|---|
| SCAI-001 | 20 mg | 20 mg | 20 mg | 20 mg | 20 mg | 10 mg |
| Castor oil | 20 mg | 20 mg | 20 mg | 20 mg | 20 mg | 20 mg |
| Polysorbate 80 | 80 mg | 80 mg | 80 mg | 80 mg | 80 mg | 80 mg |
| Glycerin | 2.4 g | 2.4 g | 2.4 g | 2.4 g | 2.4 g | 2.4 g |
| Carbomer | 50 mg | 40 mg | 30 mg | 20 mg | 10 mg | 20 mg |
| Sodium hydroxide | q.s. | q.s. | q.s. | q.s. | q.s. | q.s. |
| Purified water | q.s. | q.s. | q.s. | q.s. | q.s. | q.s. |
| Final amount | 100 mL | 100 mL | 100 mL | 100 mL | 100 mL | 100 mL |

### 2) Evaluation results

After preparing the eye drops, the evaluation results are shown in Table 4. After preparation, no precipitation occurred in any of the experiments using Compositions 1 to 6, and all were confirmed to have a transparent appearance.

The zeta measurement values varied depending on the amount of carbomer, and it was confirmed that as the amount of carbomer increased, the zeta results became unstable and the viscosity of the sample increased rapidly.

Among these, Composition 4 was confirmed to be a composition that is easy to produce and can ensure stability.

**[Table 4]**

| | Composition 1 | Composition 2 | Composi tion 3 | Composi tion 4 | Composi tion 5 | Composi tion 6 |
|---|---|---|---|---|---|---|
| pH | 7.52 | 7.57 | 7.52 | 7.49 | 7.52 | 7.52 |
| Zeta (mV) | -31.32.13 | -26.8 | -46.0 | -52.3 | -61.3 | -52.3 |
| | 30.2 | 4.69 | -15.4 | -28.8 | -28.6 | |
| | | 35.1 | 16.1 | -85.2 | | |
| Others | Bubble generation | Bubble generation | - | - | - | - |

### 3) Clinical Sample Production

Stability samples and clinical samples were prepared through a contract manufacturer capable of producing disposable eye drops, and the contents are summarized in Table 5 below.

**[Table 5]**

| | Stability sample | | Clinical sample | |
|---|---|---|---|---|
| Preparation number | 22Jun01 | 22Jun02 | 22K04 | 22K05 |
| Preparation date | 2022.06.08 | 2022.06.09 | 2022.11.21 | 2022.11.22 |
| Production unit | 40 L | 40 L | 40 L | 40 L |
| Packaging unit | 0.4 mL/70 tubes/Box | 0.4 mL/70 tubes/Box | 0.4 mL/70 tubes/Box | 0.4 mL/70 tubes/Box |

### Test Example 1. Stability test for eye-drop composition containing cyclosporine nanomolecular association(SCAI-001)

### 1. Raw material

SCAI-001 is a cold storage drug, and stability tests were conducted on one manufacturing unit (Batch. SCAI-22E01) under accelerated and long-term storage conditions. For cold storage medicines, the accelerated test was conducted at a temperature of 25°C±2°C and a humidity of 70%±5%, and the long-term storage test was conducted at a temperature of 5°C±3°C. Stability was confirmed at the initial, 1-month, 2-month, 3-month, and 6-month time points. The results of the stability test are as follows.

### 1.1 Long-term storage condition

Test criteria and results for long-term storage condition are shown in Table 6 below. The stability test results showed that all test items met the criteria during the long-term storage condition of 6 months, and no changes over time were observed, indicating that the product was stable.

**[Table 6]**

| Test item | Test criteria | Initial | Long term 1 month | Long term 2 months | Long term 3 months | Long term 6 months |
|---|---|---|---|---|---|---|
| Appearance | White, translucent liquid | Fit | Fit | Fit | Fit | Fit |
| Verification test | Main peak retention time of standard solution and test solution is same | Fit | Fit | Fit | Fit | Fit |
| Related substance | Individual: 0.7% or less | 0.31 | 0.29 | 0.23 | 0.22 | 0.22 |
| | Total related substance: 1.5% or less | 0.74 | 0.73 | 0.95 | 0.85 | 0.73 |
| Residual solvent | Ethanol 5000ppm or less | 13.6 | 16.7 | 24.5 | 35.5 | 12.59 |
| Quantitative method | 0.9 ~ 1.1mg/mL | 1.00 | 1.01 | 1.01 | 1.01 | 1.01 |

### 1.2 Accelerated condition

Test criteria and results for accelerated condition are shown in Table 7 below. The stability test results showed that the total amount of related substances increased by approximately 0.6% compared to the initial amount during the accelerated condition of 6 months, but met the criteria, and no changes over time were observed, indicating that the product was stable.

**[Table 7]**

| Test item | Test criteria | Initial | Accelerat ion 1 month | Accelerat ion 2 months | Accelerat ion 3 months | Accelerat ion 6 months |
|---|---|---|---|---|---|---|
| Appearance | White, translucent liquid | Fit | Fit | Fit | Fit | Fit |
| Verification test | Main peak retention time of standard solution and test solution is same | Fit | Fit | Fit | Fit | Fit |
| Related substance | Individual : 0.7% or less | 0.31 | 0.29 | 0.25 | 0.22 | 0.23 |
| | Total related substance: 1.5% or less | 0.74 | 0.72 | 1.03 | 1.02 | 1.30 |
| Residual solvent | Ethanol 5000ppm or less | 13.6 | 20.6 | 23.6 | 35.4 | 11.30 |
| Quantitative method | 0.9 ~ 1.1mg/mL | 1.00 | 1.00 | 1.00 | 1.00 | 1.01 |

2. Finished drug: As the finished drug, Composition 4, a 0.02% composition for eye drops, and Composition 6, a 0.01% composition for eye drops, manufactured in Example 3, were used, and a stability test was conducted on one batch per dose for research purposes to confirm the stability as a drug to be stored at room temperature. Stability test on a drug packaged in LDPE material and stored at room temperature is conducted under accelerated conditions (40°C±2°C, humidity 25%±5%), intermediate conditions (30°C±2°C, 65%±5%), and long-term storage conditions (25°C±2°C, 40%±5%) . The stability test will be conducted for 6 months under accelerated conditions, for 12 months under intermediate conditions, and for 24 months under long-term storage, and stability for up to 6 months has currently been confirmed.

### 2.1 Stability test results of 0.02% composition for eye drops (Composition 4)

### 2.1.1 Long-term storage condition

The long-term storage criteria and results are shown in Table 8. The stability test results showed that all test items met the criteria for 6 months of long-term storage condition, and no changes over time were observed, indicating that the product was stable.

**[Table 8]**

| Test item | Test criteria | Initial | Long term 1 month | Long term 3 months | Long term 6 months |
|---|---|---|---|---|---|
| Appearance | Eye drops containing a colorless or light white transparent liquid in a colorless transparent plastic container | Fit | Fit | Fit | Fit |
| Verification test | When tested according to content test, test solution and standard solution show same retention time | Fit | Fit | Fit | Fit |
| pH | 6.0 ~ 8.0 | 7.03 | 7.03 | 6.81 | 6.70 |
| Osmotic pressure | 240 ~ 300mOsmol/kg | 276 | 275 | 272 | 271 |
| Mass capacity | Individual: 9mL or more | 10.5 | 10.0 | 10.0 | 10.0 |
| | Average: 10mL or more | 10.7 | 10.6 | 10.6 | 10.5 |
| Content | 90.0 ~ 110.0% | 105.74 | 104.82 | 105.78 | 105.41 |
| Insoluble matter | Clear, no foreign matter easily observed | Fit | Fit | Fit | Fit |
| Insoluble fine particles | 10µm or more: 50 or less | 21.67 | 31.07 | 27.67 | 14.67 |
| | 25µm or more: 5 or less | 1.40 | 0. 93 | 0.47 | 0.33 |
| | 50µm or more: 2 or less | 0.07 | 0.20 | 0.07 | 0.00 |

### 2.1.2 Intermediate test condition

The intermediate test criteria and results are shown in Table 9. The stability test results showed that the content was within the criteria for 6 months under the intermediate conditions, but a significant change was observed with a decrease of approximately 10% compared to the initial content. Other items met the criteria.

**[Table 9]**

| Test item | Test criteria | Initial | Intermedia te 1 month | Intermedia te 3 months | Intermedia te 6 months |
|---|---|---|---|---|---|
| Appearance | Eye drops containing a colorless or light white transparent liquid in a colorless transparent plastic container | Fit | Fit | Fit | Fit |
| Verification test | When tested according to content test, test solution and standard solution show same retention time | Fit | Fit | Fit | Fit |
| pH | 6.0 ~ 8.0 | 7.03 | 6. 93 | 6.62 | 6.31 |
| Osmotic pressure | 240 ~ 300mOsmol/kg | 276 | 275 | 271 | 272 |
| Mass capacity | Individual: 9mL or more | 10.5 | 10.0 | 10.0 | 10.0 |
| | Average: 10mL or more | 10.7 | 10.6 | 10.5 | 10.6 |
| Content | 90.0 ~ 110.0% | 105.74 | 104.77 | 103.57 | 95.61 |
| Insoluble matter | Clear, no foreign matter easily observed | Fit | Fit | Fit | Fit |
| Insoluble fine particles | 10µm or more: 50 or less | 21.67 | 28.73 | 20.00 | 25.87 |
| | 25µm or more: 5 or less | 1.40 | 1.47 | 0.53 | 0.27 |
| | 50µm or more: 2 or less | 0.07 | 0.27 | 0.27 | 0.13 |

### 2.1.3 Accelerated test condition

Accelerated test criteria and results are shown in Table 10. As a result of the stability test, significant changes were observed in the content results for 6 months under the accelerated test conditions. It decreased by approximately 20% compared to the initial test results and did not meet the criteria.

**[Table 10]**

| Test item | Test criteria | Initial | Acceler ation 1 month | Acceler ation 3 months | Acceler ation 6 months |
|---|---|---|---|---|---|
| Appearance | Eye drops containing a colorless or light white transparent liquid in a colorless transparent plastic container | Fit | Fit | Fit | Fit |
| Verification test | When tested according to content test, test solution and standard solution show same retention time | Fit | Fit | Fit | Fit |
| pH | 6.0 ~ 8.0 | 7.03 | 7.09 | 7.13 | 7.08 |
| Osmotic pressure | 240 ~ 300mOsmol/kg | 276 | 276 | 273 | 275 |
| Mass capacity | Individual: 9mL or more | 10.5 | 10.0 | 10.0 | 10.0 |
| | Average: 10mL or more | 10.7 | 10.6 | 10.5 | 10.5 |
| Content | 90.0 ~ 110.0% | 105.74 | 101.69 | 97.93 | 84.07 |
| Insoluble matter | Clear, no foreign matter easily observed | Fit | Fit | Fit | Fit |
| Insoluble fine particles | 10µm or more: 50 or less | 21.37 | 32.47 | 65.20 | 16.13 |
| | 25µm or more: 5 or less | 1.40 | 0.13 | 4.47 | 0.40 |
| | 50µm or more: 2 or less | 0.07 | 0.00 | 0.73 | 0.00 |

### 2.2 Stability test results of 0.01% composition for eye drops (Composition 6)

### 2.2.1 Long-term storage condition

The long-term storage criteria and results are shown in Table 11. The stability test results showed that all test items met the criteria for 6 months of long-term storage condition, and no changes over time were observed, indicating that the product was stable.

**[Table 11]**

| Test item | Test criteria | Initial | Long term 1 month | Long term 3 months | Long term 6 months |
|---|---|---|---|---|---|
| Appearance | Eye drops containing a colorless or light white transparent liquid in a colorless transparent plastic container | Fit | Fit | Fit | Fit |
| Verification test | When tested according to content test, test solution and standard solution show same retention time | Fit | Fit | Fit | Fit |
| pH | 6.0 ~ 8.0 | 7.17 | 7.08 | 6. 93 | 6.77 |
| Osmotic pressure | 240 ~ 300mOsmol/kg | 274 | 275 | 270 | 271 |
| Mass capacity | Individual: 9mL or more | 10.0 | 10.0 | 10.0 | 10.0 |
| | Average: 10mL or more | 10.5 | 10.6 | 10.5 | 10.6 |
| Content | 90.0 ~ 110.0% | 104.34 | 105.07 | 104.33 | 106.13 |
| Insoluble matter | Clear, no foreign matter easily observed | Fit | Fit | Fit | Fit |
| Insoluble fine particles | 10µm or more: 50 or less | 43.47 | 38.27 | 32.67 | 30.33 |
| | 25µm or more: 5 or less | 1.20 | 1.20 | 0.60 | 0.80 |
| | 50µm or more: 2 or less | 0.33 | 0.07 | 0.00 | 0.00 |

### 2.2.2 Intermediate test condition

The intermediate test criteria and results are shown in Table 12. The stability test results showed that all test items met the criteria for 6 months under the intermediate condition, and no changes over time were observed, indicating that the test was stable.

**[Table 12]**

| Test item | Test criteria | Initial | Intermedi ate 1 month | Intermedi ate 3 months | Intermedi ate 6 months |
|---|---|---|---|---|---|
| Appearance | Eye drops containing a colorless or light white transparent liquid in a colorless transparent plastic container | Fit | Fit | Fit | Fit |
| Verification test | When tested according to content test, test solution and standard solution show same retention time | Fit | Fit | Fit | Fit |
| pH | 6.0 ~ 8.0 | 7.17 | 7.09 | 6. 93 | 7.04 |
| Osmotic pressure | 240 ~ 300mOsmol/kg | 274 | 274 | 271 | 270 |
| Mass capacity | Individual: 9mL or more | 10.0 | 10.0 | 10.0 | 10.0 |
| | Average: 10mL or more | 10.5 | 10.7 | 10.6 | 10.7 |
| Content | 90.0 ~ 110.0% | 104.34 | 104.97 | 103.73 | 104.89 |
| Insoluble matter | Clear, no foreign matter easily observed | Fit | Fit | Fit | Fit |
| Insoluble fine particles | 10µm or more: 50 or less | 43.47 | 43.87 | 42.33 | 38.40 |
| | 25µm or more: 5 or less | 1.20 | 0.80 | 1.20 | 1.13 |
| | 50µm or more: 2 or less | 0.33 | 0.20 | 0.07 | 0.07 |

### 2.2.3 Accelerated test condition

Accelerated test criteria and results are shown in Table 13. As a result of the stability test, the content result for 6 months under the accelerated test conditions was within the criteria, but a significant change was observed with a decrease of approximately 8% compared to the initial test results.

**[Table 13]**

| Test item | Test criteria | Initial | Accelerat ion 1 month | Accelerat ion 3 month | Accelerat ion 6 month |
|---|---|---|---|---|---|
| Appearance | Eye drops containing a colorless or light white transparent liquid in a colorless transparent plastic container | Fit | Fit | Fit | Fit |
| Verification test | When tested according to content test, test solution and standard solution show same retention time | Fit | Fit | Fit | Fit |
| pH | 6.0 ~ 8.0 | 7.17 | 7.16 | 7.11 | 6.95 |
| Osmotic pressure | 240 ~ 300mOsmol/kg | 274 | 273 | 271 | 272 |
| Mass capacity | Individual: 9mL or more | 10.0 | 10.0 | 10.0 | 10.0 |
| | Average: 10mL or more | 10.5 | 10.5 | 10.5 | 10.5 |
| Content | 90.0 ~ 110.0% | 104.34 | 104.16 | 97.12 | 96.43 |
| Insoluble matter | Clear, no foreign matter easily observed | Fit | Fit | Fit | Fit |
| Insoluble fine particles | 10µm or more: 50 or less | 43.47 | 43.93 | 60.53 | 27.47 |
| | 25µm or more: 5 or less | 1.20 | 1.40 | 2.47 | 0.40 |
| | 50µm or more: 2 or less | 0.33 | 0.13 | 0.47 | 0.07 |

### Test Example 2. PK/PD test of eye-drop composition containing cyclosporine nanomolecular association (SCAI-001)

Composition 4, a 0.02% composition for eye drops, and Composition 6, a 0.01% composition for eye drops prepared in Example 3, and an eye drop control preparation (Restasis, 0.05%) as Comparative Example 1 were used, and the pharmacokinetic distribution in ocular tissue and blood was evaluated using NZW rabbits after single or multiple eye drop administration (at 12-hour intervals for 7 days). After dropping 50 µL of the control and test preparations into the left and right eyes of rabbits, respectively, the animals were euthanized at each time point and blood and various tissue samples were collected. For single-eye drop tests, samples were collected immediately after eye drop, 30 minutes later, and 1, 3, 6, 12, 24, 48, and 72 hours later. For multiple eye drop tests, samples were collected 1, 6, 24, and 72 hours after the last eye drop on day 7. Blood and nine ocular tissue samples (cornea, conjunctiva, aqueous humor, lens, iris, vitreous humor, sclera, choroid, and retina) were collected from two rabbits at each sampling time point. Drug components in each sample were extracted and their concentrations were analyzed using a Sciex QTRAP6500 mass spectrometer.

Using analysis of Phoenix^{®} WinNonlin^{®} software, the main pharmacokinetic parameters were calculated for the results of quantitative analysis of animal experiments, and the absorption, distribution, and elimination patterns in the eye over time after eye drop of Example of the present invention were compared with those of the control preparation (Restasis).

The control preparation Restasis (0.05%) and the 0.02% composition for eye drops, Composition 4, and the 0.01% composition for eye drops, Composition 6, prepared in Example 3 of the present invention were administered to the rabbit eye once or repeatedly, and then the distribution within the eye was confirmed. The pharmacodynamics of Example and Comparative Example were compared and evaluated to determine how much of the drug active ingredient, Cyclosporine A, was delivered to the blood and ocular tissues over time after eye drop administration. The LC/MS analysis method used in the analysis of this Example and Comparative Example was first verified, and then sample analysis was performed under consistent analysis conditions. For quantitative analysis, Cyclosporine A standard was diluted by concentration to prepare a sample standard solution, and then analyzed after organic solvent extraction or solid phase extraction (SPE). Then, the drug concentration in whole blood and ocular tissue was calculated using a calibration curve for each biological sample. The results are summarized in Tables 14 and 15 and FIGS. 1 and 2.

Table 14 below reports the blood and tissue PK parameters of SCAI-001 (0.01%, 0.02%) and Restasis (0.05%) in a single-dose eye drop administration test.

**[Table 14]**

| Single | Group | Dose (µg/kg) | Tₘₐₓ (h) | Cₘₐₓ (ng/mL or ng/g) | AUCₗₐₛₜ (h*ng/mL or h*ng/g) |
|---|---|---|---|---|---|
| Cornea | G1 (SCAI-001, 0.01%) | 4 | 6.0 | 423.88 (± 85.26) | 14501.73 (± 2246.94) |
| | G2 (SCAI-001, 0.02%) | 8 | 3.0 | 664.50 (± 110.02) | 21979.46 (± 1503.20) |
| | G3 (Restasis, 0.05%) | 20 | 12.0 | 388.75 (± 69.18) | 13257.85 (± 1469.71) |
| Conjunctiva | G1 (SCAI-001, 0.01%) | 4 | 0.5 | 1580.25 (± 329.75) | 7139.55 (± 587.12) |
| | G2 (SCAI-001, 0.02%) | 8 | 0.5 | 1940.00 (± 298.27) | 9437.89 (± 615.89) |
| | G3 (Restasis, 0.05%) | 20 | 0.5 | 896.25 (± 131.84) | 6855.43 (± 520.13) |
| AH | G1 (SCAI-001, 0.01%) | 4 | 0.5 | 0.53 | - |
| | G2 (SCAI-001, 0.02%) | 8 | 6.0 | 0.59 | - |
| | G3 (Restasis, 0.05%) | 20 | - | - | - |
| VH | G1 (SCAI-001, 0.01%) | 4 | - | - | - |
| | G2 (SCAI-001, 0.02%) | 8 | - | - | - |
| | G3 (Restasis, 0.05%) | 20 | - | - | - |
| Iris | G1 (SCAI-001, 0.01%) | 4 | 24.0 | 12.11 (± 2.53) | 543.90 (± 58.74) |
| | G2 (SCAI-001, 0.02%) | 8 | 24.0 | 7.19 (± 1.32) | 409.43 (± 40.63) |
| | G3 (Restasis, 0.05%) | 20 | 12.0 | 4.15 (± 1.68) | 159.05 (± 33.81) |
| Lens | G1 (SCAI-001, 0.01%) | 4 | - | - | - |
| | G2 (SCAI-001, 0.02%) | 8 | - | - | - |
| | G3 (Restasis, 0.05%) | 20 | - | - | - |
| Sclera | G1 (SCAI-001, 0.01%) | 4 | 1.0 | 8.73 (± 1.48) | 233.99 (± 34.33) |
| | G2 (SCAI-001, 0.02%) | 8 | 3.0 | 13.18 (± 2.38) | 324.32 (± 44.26) |
| | G3 (Restasis, 0.05%) | 20 | 12.0 | 10.19 (± 2.62) | 232.10 (± 38.90) |
| Choroid | G1 (SCAI-001, 0.01%) | 4 | 0.5 | 5.61 | 2.42 (± 1.85) |
| | G2 (SCAI-001, 0.02%) | 8 | 3.0 | 2.39 (± 1.44) | 8.62 (± 4.47) |
| | G3 (Restasis, 0.05%) | 20 | 12.0 | 4.79 | 8.70 (± 6.25) |
| Retina | G1 (SCAI-001, 0.01%) | 4 | - | - | - |
| | G2 (SCAI-001, 0.02%) | 8 | 3.0 | 1.06 (± 0.61) | 3.98 (± 2.02) |
| | G3 (Restasis, 0.05%) | 20 | - | - | - |
| Blood | G1 (SCAI-001, 0.01%) | 4 | 1.0 | 0.56 | - |
| | G2 (SCAI-001, 0.02%) | 8 | 1.0 | 1.28 (± 0.82) | 2.09 (± 1.08) |
| | G3 (Restasis, 0.05%) | 20 | - | - | - |

Table 15 below shows the blood and tissue PK parameters and accumulation rates compared to single eye drop administration of SCAI-001 (0.01%, 0.02%) and Restasis (0.05%) in multiple eye drop administration studies.

**[Table 15]**

| Multiple | Group | Dose (µg/eye) | Tₘₐₓ (h) | Cₘₐₓ (ng/mL or ng/g) | AUCₗₐₛₜ (h*ng/mL or h*ng/g) | Accumulat ion (%) |
|---|---|---|---|---|---|---|
| Cornea | G1 (SCAI-001, 0.01%) | 4 | 6 | 1812.50 (± 315.47) | 77677.25 (± 7464.07) | 535.64% |
| | G2 (SCAI-001, 0.02%) | 8 | 6 | 1822.50 (± 142.27) | 85080.00 (± 7013.31) | 387.09% |
| | G3 (Restasis, 0.05%) | 20 | 6 | 891.00 (± 84.04) | 42546.00 (± 6737.86) | 320.91% |
| Conjunctiva | G1 (SCAI-001, 0.01%) | 4 | 1 | 719.25 (± 174.86) | 5182.16 (± 972.60) | 72.58% |
| | G2 (SCAI-001, 0.02%) | 8 | 1 | 1101.50 (± 340.87) | 6865.28 (± 1194.88) | 72.74% |
| | G3 (Restasis, 0.05%) | 20 | 1 | 795.75 (± 63.19) | 6379.77 (± 1384.66) | 93.06% |
| AH | G1 (SCAI-001, 0.01%) | 4 | 6 | 1.49 (± 0.42) | 25.55 (± 5.74) | - |
| | G2 (SCAI-001, 0.02%) | 8 | 1 | 1.55 (± 0.09) | 71.12 (± 10.20) | - |
| | G3 (Restasis, 0.05%) | 20 | 1 | 0.61 (± 0.03) | 5.73 (± 2.04) | - |
| VH | G1 (SCAI-001, 0.01%) | 4 | 1 | 0.41 (± 0.09) | 0.10 (± 0.36) | - |
| | G2 (SCAI-001, 0.02%) | 8 | 1 | 0.51 | - | - |
| | G3 (Restasis, 0.05%) | 20 | 6 | 0.38 | - | - |
| Iris | G1 (SCAI-001, 0.01%) | 4 | 1 | 49.56 (± 6.87) | 2010.22 (± 84.86) | 369.59% |
| | G2 (SCAI-001, 0.02%) | 8 | 1 | 50.83 (± 5.90) | 2770.68 (± 172.23) | 676.71% |
| | G3 (Restasis, 0.05%) | 20 | 1 | 34.43 (± 2.96) | 1445.59 (± 83.98) | 908.87% |
| Lens | G1 (SCAI-001, 0.01%) | 4 | 24 | 11.78 (± 1.23) | 772.94 (± 43.57) | - |
| | G2 (SCAI-001, 0.02%) | 8 | 72 | 14.25 (± 1.37) | 836.40 (± 104.46) | - |
| | G3 (Restasis, 0.05%) | 20 | 72 | 7.29 (± 1.52) | 420.23 (± 46.26) | - |
| Sclera | G1 (SCAI-001, 0.01%) | 4 | 1 | 23.69 (± 3.00) | 605.86 (± 79.34) | 258.92% |
| | G2 (SCAI-001, 0.02%) | 8 | 1 | 33.98 (± 6.55) | 1121.40 (± 160.34) | 345.77% |
| | G3 (Restasis, 0.05%) | 20 | 1 | 29.69 (± 8.85) | 837.37 (± 109.62) | 360.78% |
| Choroid | G1 (SCAI-001, 0.01%) | 4 | 1 | 2.14 (± 1.24) | 30.32 (± 17.99) | 1255.43% |
| | G2 (SCAI-001, 0.02%) | 8 | 6 | 3.93 (± 1.47) | 85.85 (± 20.19) | 995.84% |
| | G3 (Restasis, 0.05%) | 20 | 1 | 3.03 (± 1.02) | 15.18 (± 4.69) | 173.39% |
| Retina | G1 (SCAI-001, 0.01%) | 4 | 1 | 2.31 | - | - |
| | G2 (SCAI-001, 0.02%) | 8 | 6 | 1.72 (± 1.04) | 5.82 (± 3.01) | 146.30% |
| | G3 (Restasis, 0.05%) | 20 | - | - | - | - |
| Blood | G1 (SCAI-001, 0.01%) | 4 | 1 | 0.45 (± 0.1) | 0.22 (± 0.29) | - |
| | G2 (SCAI-001, 0.02%) | 8 | 1 | 0.38 (± 0.03) | 0.19 (± 0.09) | 9.12% |
| | G3 (Restasis, 0.05%) | 20 | 1 | 0.38 | 0.10 (± 0.57) | - |

In a single-dose eye drop administration test, the drug exposure analyzed based on AUClast was found to be highest in the cornea and conjunctiva, which are the drug targets, for both the control and test preparations, and the specific exposure amounts were as follows. (Composition 6, a 0.01% composition for eye drops prepared in Example 3, 14501.73 h*ng/g and 7139.55 h*ng/g; Composition 4, a 0.02% composition for eye drops prepared in Example 3, 21979.46 h*ng/g and 9437.89 h*ng/g; as Comparative Example 1, an eye drop control preparation (Restasis, 0.05%), 13257.85 h*ng/g and 6855.43 h*ng/g)

Tissues other than cornea and conjunctiva showed high exposure amounts in the order of iris, sclera, choroid, retina, and AH (aqueous humor). On the other hand, concentrations below the lower limit of quantitation were detected in VH (vitreous humor) and lens.

In particular, Example was confirmed to have higher exposure amount to cornea and conjunctiva after a single administration, despite a lower eye drop dose compared to the control preparation (Restasis), which is a comparative example (When dose normalized by AUClast, the exposure amount in cornea and conjunctiva was 5.47 times and 5.21 times higher for Composition 6, a 0.01% composition for eye drops prepared in Example 3, and 3.98 times and 3.44 times higher for Composition 4, a 0.02% eye drop composition manufactured in Example 3, compared to Restasis (0.05%.).

In whole blood, the Example reached Cmax within 1 hour and was detected at concentrations below the lower limit of quantitation at most time points except Cmax.

In multiple eye drop administration tests, drug exposure amount analyzed based on AUClast was found to be highest in the cornea for both the Comparative Example and the Example samples (Composition 6, a 0.01% composition for eye drops manufactured in Example 3, 77677.25 h*ng/g; Composition 4, a 0.02% composition for eye drops prepared in Example 3, 85080 h*ng/g; as Comparative Example 1, an eye drop control preparation (Restasis, 0.05%), 42546 h*ng/g), followed by the conjunctiva, iris, sclera, lens, choroid, AH, and retina. In VH, it was detected at a concentration below the lower limit of quantitation. In the case of multiple eye drop administration tests, the exposure amount of cornea and conjunctiva was also confirmed to be higher in the Example than in the Comparative Example, even though the dose was lower (When dose normalized by AUClast, the exposure amount in cornea and conjunctiva was 9.13 times and 4.06 times higher for Composition 6, a 0.01% composition for eye drops, and 5.00 times and 2.69 times higher for Composition 4, a 0.02% composition for eye drops). In whole blood, the test preparation was confirmed to reach Cmax within 1 hour, and was detected at concentrations below the lower limit of quantitation at most time points except Cmax. In multiple eye drop administration tests, drug accumulation was confirmed in all biological samples except conjunctiva and whole blood, and the accumulation rate ranged from 72.58 to 1255.43%.

In the case of Composition 6, a 0.01% composition for eye drops prepared in Example 3, it was distributed in the cornea, conjunctiva, iris, and choroid at a higher concentration than that of Comparative Example 1, the eye drop control preparation (Restasis, 0.05%), after administration, and in the case of Composition 4, a 0.02% composition for eye drops prepared in Example 3, it was distributed in all tissues at a higher concentration than that of Comparative Example 1. This indicates that the active ingredient of the drug is delivered more efficiently to the ocular target tissue at a lower dose in the Example than in the Comparative Example. Additionally, the exposure amount in cornea and conjunctiva accounted for 96.52 to 98.05% of the total amount of Cyclosporine A detected in the whole tissue in a single eye drop administration test and 94.73 to 96.01% in a multiple eye drop administration test.

In conclusion, the Example of the present invention showed that most of the drug after administration was present in the target tissues, cornea and conjunctiva, and was delivered to the target tissue more efficiently at a lower concentration than the Comparative Example. In addition, the amount of blood releasing out to the whole body other than the target tissue, the eye tissue, was low at 0.02% or less of the total eye drop dosage for both single and multiple eye drop administrations.

## Claims

1. A molecular association in which cyclosporine is physically bound, wherein molecular association in the composition has an aggregated structure when the molecular association is prepared as a composition containing water.

2. The molecular association of claim 1, wherein the molecular association has an average particle diameter of 1.0 to 10 nm or less.

3. The molecular association of claim 1, wherein the total amount of related substances is 1.5% or less of the initial amount after 6 months under long-term storage conditions at a temperature of 5°C±3°C.

4. The molecular association of claim 1, wherein the total amount of related substances is 1.5% or less of the initial amount after 6 months under accelerated conditions of 25°C±2°C/70%±5%.

5. A composition for preparing an eye drops comprising the molecular association of claim 1, a surfactant, a solubilizer, and purified water.

6. The composition for preparing an eye drops of claim 5, wherein the molecular association is contained in an amount of 0.001 to 0.04 wt% based on the total content of the composition.

7. The composition for preparing an eye drops of claim 5, wherein the molecular association is contained in an amount of 0.001 to 0.02 wt% based on the total content of the composition.

8. The composition for preparing an eye drops of claim 5, wherein the surfactant is contained in an amount of 0.001 to 0.5 wt% based on the total content of the composition.

9. The composition for preparing an eye drops of claim 5, wherein the surfactant is contained in an amount of 0.01 to 0.1 wt% based on the total content of the composition.

10. The composition for preparing an eye drops of claim 5, wherein the solubilizer is contained in an amount of 0.001 to 0.1 wt% based on the total content of the composition.

11. The composition for preparing an eye drops of claim 5, wherein the surfactant is at least one selected from the group consisting of: Polysorbate 20, Polysorbate 40, Polysorbate 60. Polysorbate 80, alcohol, amine oxide, block polymer, carboxylate alcohol, alkylphenol ethoxylate carboxylic acid/fatty acid, ethoxylate alcohol, ethoxylate alkylphenol, ethoxylate aryl phenol, ethoxylated fatty acid, ethoxylate, fatty acid ester or oil, fatty acid ester, fatty acid methyl ester ethoxylate, glycerol ester, glycol ester, lanolin derivatives, lecithin, lecithin derivatives, lignin, lignin derivatives, methyl ester, monoglyceride, monoglyceride derivatives, polyethylene glycol, polymeric surfactant, propoxylate and ethoxylate fatty acids, alcohol or alkyl phenol, protein derived surfactant, sarcosine derivatives, sorbitan derivatives, sucrose, sucrose derivatives, glucose esters, and glucose ester derivatives.

12. The composition for preparing an eye drops of claim 5, wherein the solubilizer is at least one selected from the group consisting of: castor oil, canola oil, alkyl (C12-15) benzoate, almond oil, aluminum monostearate, cetostearyl alcohol, cholesterol, coconut oil, cyclomethicone, dimethicone, ethylene glycol stearate, glycerin, glyceryl monooleate, glyceryl monostearate, isopropyl myristate, isopropyl palmitate, hydrogenated lanolin, lecithin, mineral oil, myristyl alcohol, octyldodecanol, oleyl alcohol, oleyl oleate, petrolactum, polydecane, propylene glycol dilaurate, propylene glycol monolaurate, safflower oil, soybean oil, sunflower oil, wax, xylitol and zinc acetate.

13. An eye-drop composition further comprising at least one selected from the group consisting of a viscosity adjusting agent, an osmotic pressure adjusting agent and a pH adjusting agent in the composition for preparing an eye drops of claim 5.

14. The eye-drop composition of claim 13, wherein the viscosity adjusting agent is contained in an amount of 0.001 to 0.5 wt% based on the total content of the composition.

15. The eye-drop composition of claim 13, wherein the osmotic pressure adjusting agent is contained in an amount of 0.5 to 10.0 wt% based on the total content of the composition.

16. The eye-drop composition of claim 13, wherein the content of the viscosity adjusting agent is 0.5 to 2.5 times the content of the molecular association.

17. The eye-drop composition of claim 13, wherein the viscosity adjusting agent is at least one selected from the group consisting of: acacia, agal, aramic acid, alginic acid, aluminum monostearate, attapulgite, bentonite, carbomer, carboxymethylcellulose calcium, carboxymethylcellulose sodium, carmellrose, carrageenan, cellulose, microstalline, cetostearyl alcohol, chitosan, corn syrup, cyclomethicone, dextrin, egg phospholipids, ethyl cellulose, gelatin, magnesium aluminum silicate, maltitol solution, maltodextrin, medium-chain triglycerides, methylcellulose, pectin, polycarbophil, polydextrose, polyethylene oxide, polyvinyl alcohol, potassium alginate, povidone, propylene glycol alginate, pullulan, silica dental-type, silica hydrophobic colloidal, silicon dioxide, silicon dioxide colloidal, sodium alginate, sorbitan monolaurate, sorbitan monopalmitate, sorbitan monostearate, sorbitan sesquioleate, sorbitan trioleate, starch corn, starch hydroxypropyl corn, starch pregelatinized hydroxypropyl corn, starch pea, starch hydroxypropyl pea, starch pregelatinized hydroxypropyl pea, starch potato, gellan gum, glyceric behenate, glyceric dibehenate, guar gum, hydroxyethyl cellulose, hydroxypropyl cellulose, hydromellose, isomalt, alpha-lactalbumin, kaolin, starch hydroxypropyl potato, starch pregelatinized hydroxypropyl potato, starch tapioca, starch wheat, sucrose, sucrose palmitate, tragacanth, vitamin E propylene glycol succinate, and xanthan gum.

18. The eye-drop composition of claim 13, wherein the osmotic pressure adjusting agent is at least one selected from the group consisting of glycerin, sorbitol, mannitol, dextrose and sucrose.

19. The eye-drop composition of claim 13, wherein the pH adjusting agent is at least one selected from the group consisting of: acetic acid, adipic acid, ammonia solution, ammonium carbonate, ammonium chloride, ammonium phosphate, boric acid, calcium carbonate, calcium hydroxide, calcium lactate, calcium phosphate, citric acid, diethanolamine, fumaric acid, glycine, hydrochloric acid, alpha-lactalbumin, lactic acid, lysine hydrochloride, malic acid, maleic acid, methionine, monoethanolamine, monosodium glutamate, nitric acid, phosphoric acid, potassium citrate, potassium hydroxide, potassium metaphosphate, potassium phosphate, propionic acid, racemethionine, sodium acetate, sodium bicarbonate, sodium borate, sodium carbonate, sodium citrate, sodium hydroxide, sodium lactate solution, sodium phosphate dibasic, sodium phosphate monobasic, succinic acid, sulfuric acid, tartaric acid and trolamine.

20. The eye-drop composition of claim 13, wherein the molecular association is contained in an amount of 0.001 to 0.04 wt% based on the total content of the composition, and the total content change for 6 months under long-term storage conditions of 25°C±2°C/40%±5% is 90.0 to 110.0%.

21. The eye-drop composition of claim 13, wherein the molecular association is contained in an amount of 0.001 to 0.04 wt% based on the total content of the composition, and the total content change for 6 months under intermediate conditions of 30°C±2°C/65%±5% is 90.0 to 110.0%.

22. The eye-drop composition of claim 13, wherein the molecular association is contained in an amount of 0.001 to 0.04 wt% based on the total content of the composition, and the total content change for 6 months under acceleration conditions of 40°Ct2°C/25%t5% is 80.0 to 110.0%.

23. The eye-drop composition of claim 13, wherein the exposure amount to cornea and conjunctiva is 3 times or more when administered to the eye.

24. The eye-drop composition of claim 13, wherein the amount of blood releasing out to the whole body other than the eye tissue is 0.02% or less of the total eye drop dosage when administered to the eye.

25. A method for preparing a molecular association in which cyclosporine is physically bound, comprising the steps of: 1) pouring a cyclosporine solution into silica and then repeatedly releasing cyclosporine; 2) filtering the released solution and then concentrating the filter filtrate through vacuum distillation; 3) filtering the concentrated residual liquid and then crystallizing it; and 4) filtering, washing, and drying the crystallized solid.
